# EUROPEAN PATENT SPECIFICATION

(11) **EP 0 380 823 B1**
(45) Date of publication and mention of the grant of the patent: **26.10.1994**
(21) Application number: 89203098.2
(22) Date of filing: 06.12.1989
(51) Int. Cl.: C12N 15/74, C12N 1/20, A23C 19/032

(54) **Genetically manipulated lactic acid bacteria and their use in the manufacture of cheese, as well as recombinant plasmids**
Genetisch modifizierte Milchsäurebakterien und ihre Verwendung bei der Herstellung von Käse und rekombinanten Plasmiden
Bactéries lactiques manipulées génétiquement et leur utilisation pour la préparation de fromage et plasmides recombinants

(30) Priority: 07.12.1988 NL 8803004
(43) Date of publication of application: 08.08.1990
(73) Proprietor: BCZ Friesland B.V., NL-8911 AK Leeuwarden (NL)
(72) Inventor: Venema, Gerhardus, NL-9751 NN Haren (NL); Kok, Jan, NL-9715 LR Groningen (NL); van der Vossen, Josephus Mauritius Bernardus Maria, NL-3702 AP Zeist (NL); van de Guchte, Maarten, NL-9753 AM Haren (NL)
(74) Representative: Smulders, Theodorus A.H.J., Ir.

(56) References cited:
- NL-A- 8 700 890
- NIZO NIEUWS, no. 10, 1987, pages 17-20, Ede, NL; J. STADHOUDERS et al.: "Het gebruik van lysozym ter voorkoming van boterzuurgisting in Goudse kaas"
- PROTEIN ENGINEERING, vol. 1, no. 4, 1987, pages 327-332, IRL Press Ltd, Oxford, GB, T. MIKI et al.: "Construction of a plasmid vector for the regulatable high level expression of eukaryotic genes in Escherichia coli: an application to overproduction of chicken lysozyme"
- MOLECULAR AND GENERAL GENETICS, vol. 182, 1981, pages 326-331, Springer Verlag, Heidelberg, DE; J. GARRETT et al.: "Cell lysis by induction of cloned lambda lysis genes"
- APPLIED AND ENVIRONMENTAL MICROBIOLOGY, vol. 55, no. 1, January 1989, pages 224-228, American Society for Microbiology, Washington DC, US; M. VAN DE GUCHTE et al.: "Construction of a lactococcal expression vector: expression of hen egg white lysozyme in Lactococcus lactis subsp. lactis"

## Description

### Field of the invention

This invention relates to genetically manipulated lactic acid bacteria, particularly of the species Lactococcus lactis as well as to the use of these bacteria in the manufacture of cheese and to recombinant plasmids capable of being used for genetic manipulation.

This invention is therefore in the field of genetic manipulation using DNA recombinant technology as well as in the field of dairy technology, particularly the manufacture of cheese.

### Background of the invention

A problem which may arise in the manufacture of cheese, e.g., the production of Gouda and Edam cheese, is the occurrence of so-called butyric acid fermentation, i.e. the occurrence of flavour deficiencies as a result of the growth of clostridia species, particularly Clostridium tyrobutyricum, spores of which may be contained in the cheese milk. Particularly winter milk is often contaminated by a significant amount of spores of these butyric acid bacteria.

Because the relevant spores easily survive pasteurization treatments, special measures are required to inhibit butyric acid fermentation in cheese. A very conventional and effective measure consists in adding nitrate to the cheese milk. From a viewpoint of health, however, objections have arisen against the presence of relatively large amounts of nitrate in food products, such as cheese. By means of the formation of nitrite, nitrate would give rise to the formation of nitrosamines which are known to be carcinogenic substances. For this reason it is forbidden in some countries to import cheese containing more than 10 mg nitrate per kg cheese.

Since 1966 it has been known that butyric acid fermentation can also be inhibited by adding lysozyme recovered from the white of hen's eggs (Pulay, 1966, XVII Int.Dairy Congr.D641). For more recent studies reference is made to Wasserfall and Teuber, Appl.Environ. Microbiol. 38, 1979, 197-199 and Stadhouders et al, NIZO-Nieuws No. 10, 1987, 17-20.

It is further known from Biochim.Biophys.Acta 615, 1980, 489-496 and from Mol.Gen.Genet.182, 1981, 326-331 that the R gene product of bacteriophage lambda is a transglycosylase having a much higher bacteriolytic activity than hen's egg white lysozyme.

For hen's egg white lysozyme attempts have meanwhile also been made to produce the enzyme by a microbiological process. Miki et al, Protein Engineering 1, 1987, 327-332 and Imoto et al, Protein Engineering 1, 1987, 333-338 described the construction of recombinant plasmids containing a gene coding for hen's egg white lysozyme and expression of the gene in Escherichia coli bacteria. It turns out that the lysozyme accumulates in this microorganism in the form of an inactive precipitate, which is attributed to the incorrect folding of the enzyme.

In the manufacture of cheese lactic acid bacteria play an important role. The flavour and structure properties of the cheese are highly determined by the employed species and strains of lactic acid bacteria.

With respect to genetic manipulation of lactic acid bacteria, such as the different species of streptococci, lactococci and lactobacilli, the recombinant DNA technology has meanwhile made considerable progress. Thus, there have already been developed transformation systems for lactic acid bacteria which are successfully used for cloning and characterizing several homologous genes. Kok et al., Appl.Environ.Microbiol.48, 1984, 726-731 described suitable vectors which owing to a special origin of replication from the Lactococcus lactis subsp.cremoris Wg2 plasmid pWV01 are capable of replication both in lactic acid bacteria and in Escherichia coli and Bacillus subtilis. Kok et al., Appl. Environ.Microbiol.54, 1988, 231-238 determined the nucleotide sequence of the cell wall proteinase gene of the Lactococcus lactis subsp. cremoris Wg2 strain and the associate expression signals, namely, a promoter and a terminator sequence. Furthermore, Van der Vossen et al., Appl.Environ.Microbiol.50, 1985, 540-542 and 53, 1987, 2452-2457, isolated and characterized signals for gene expression of chromosomal DNA of the above Wg2 strain. By means of these aids described in the literature it seems possible now to also express heterologous genes in lactic acid bacteria. Considered by itself, this is an interesting fact, because lactic acid bacteria are reckoned among the category of the "GRAS" organisms ("generally regarded as safe") and therefore seem very appropriate for safely producing commercially important proteins.

Up to now, however, only a very small number of heterologous genes have been expressed in lactic acid bacteria, especially antibiotic resistance genes for use as selection gene in different cloning vectors (De Vos, FEMS Microbiol.Rev.46, 1987, 281-295). As regards eukaryotic DNA sequences, only the expression in Lactococcus lactis subsp.lactis of the (truncated) bovine prochymosine gene has been described so far (Simons and De Vos, Proc.4th European Congress on Biotechnology 1987, 458, and Klessen et al, Mol.Gen.Genet.212, 1988, 295-300).

### Summary of the invention

This invention provides lactic acid bacteria particularly of the species Lactococcus lactis, which as a result of a genetic manipulation have acquired the power of producing lysozyme.

More in particular, this invention provides such lactic acid bacteria, which as a result of genetic manipulation have acquired the power of producing an enzyme selected from hen's egg white lysozyme and the R gene product of bacteriophage lambda.

In the strains of lactic acid bacteria hitherto realized according to the invention and having the power acquired by genetic manipulation of producing lysozyme, this power is plasmid encoded. These strains of lactic acid bacteria always contain at least one plasmid responsible for the power of producing lysozyme, said plasmid containing at least the replication origin of the Lactococcus lactis subsp.cremoris Wg2 plasmid pWV01 and a gene coding for a lysozyme under control of expression signals active in lactic acid bacteria. Some of the resulting strains have additionally acquired the power of separating the produced lysozyme. The actually realized strains are Lactococcus lactis subsp.lactis bacteria containing the plasmid pMG37, pMG38, pMG41, or pMG42. These plasmids and their construction are described in the practical part.

This invention extends to the use of such lactic acid bacteria obtained by genetic manipulation in the manufacture of cheese and to the resulting cheese.

This invention also relates to recombinant DNA in the form of plasmids capable of being used for the above genetic manipulation of lactic acid bacteria, especially a recombinant plasmid containing a replication origin active in lactic acid bacteria and a gene coding for a lysozyme under control of expression signals active in lactic acid bacteria. The gene coding for a lysozyme is preferably a gene coding for hen's egg white lysozyme or the R gene of bacteriophage lambda.

As noted above, preference is given to a recombinant plasmid containing the replication origin of the Lactococcus lactis subsp.cremoris Wg2 plasmid pWV01.

It is further preferred that the recombinant plasmid contains the terminator sequence of the proteinase gene of Lactococcus lactis subsp.cremoris Wg2.

Some embodiments of the invention relate to a recombinant plasmid containing the promoter and the signal sequence of the protease gene of Lactococcus lactis subsp.cremoris Wg2. Lactic acid bacteria containing such a plasmid are capable of separating the resulting lysozyme. Other embodiments of the invention relate to a recombinant plasmid containing promoter P32 from chromosomal DNA of Lactococcus lactis subsp.cremoris Wg2. Genetically manipulated lactic acid bacteria containing such a plasmid lack the power of separating the produced enzyme.

It is preferred that a recombinant plasmid according to the invention also contains one or more selection genes. Appropriate as such are, e.g., antibiotic resistance genes, such as a kanamycin resistance gene or an erythromycin resistance gene.

Recombinant plasmids according to the invention which in the mean time have actually been constructed are pMG36HEL, pMG36eHEL, pMG33eHEL, pMG36lambdaR and pMG33lambdaR, as well as the plasmids pMG37, pMG38, pMG41 and pMG42 that are most preferred because of their inherent properties.

Finally, this invention is also embodied in a recombinant vector, from which one of the above described recombinant plasmids according to the invention is derived by insertion of the gene coding for a lysozyme, such as the gene coding for hen's egg white lysozyme or the R gene of bacteriophage lambda. Recombinant vectors from which the above-mentioned plasmids are derived are pMG33, pMG33e, pMG36 and pMG36e.

### Brief description of the drawings

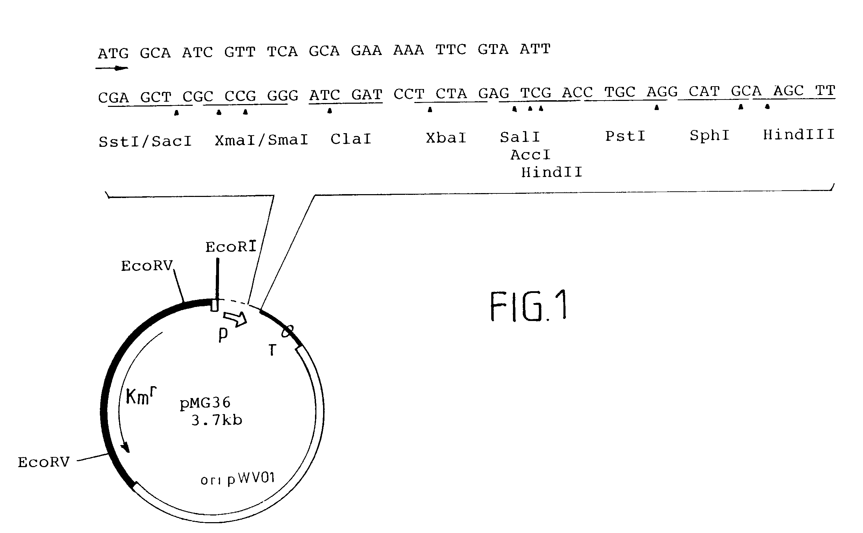

Fig. 1 maps out the expression vector pMG36. This vector has a size of 3.7 kb (kilobase) and is partly derived from the starting plasmid pGK11 (indicated by an open bar in which the replication origin of plasmid pWV01, briefly designated as ori pWV01, is present), for another part derived from the starting plasmid pPJ1 (indicated by a big solid bar in which a kanamycin resistance gene, briefly designated as Km^{r}, is present), for yet another part derived from the proteinase gene of L.lactis subsp.cremoris Wg2 (indicated by a narrow solid bar in which a transcription terminator, briefly indicated by T, is present), and for a part derived from the starting plasmid pGKV32 (indicated by a dotted line in which a promoter P, a ribosome binding site and the start of an open reading frame are present). The start of the open reading frame (indicated by an arrow) and the multiple cloning site (MCS) are shown in an elaborate form.

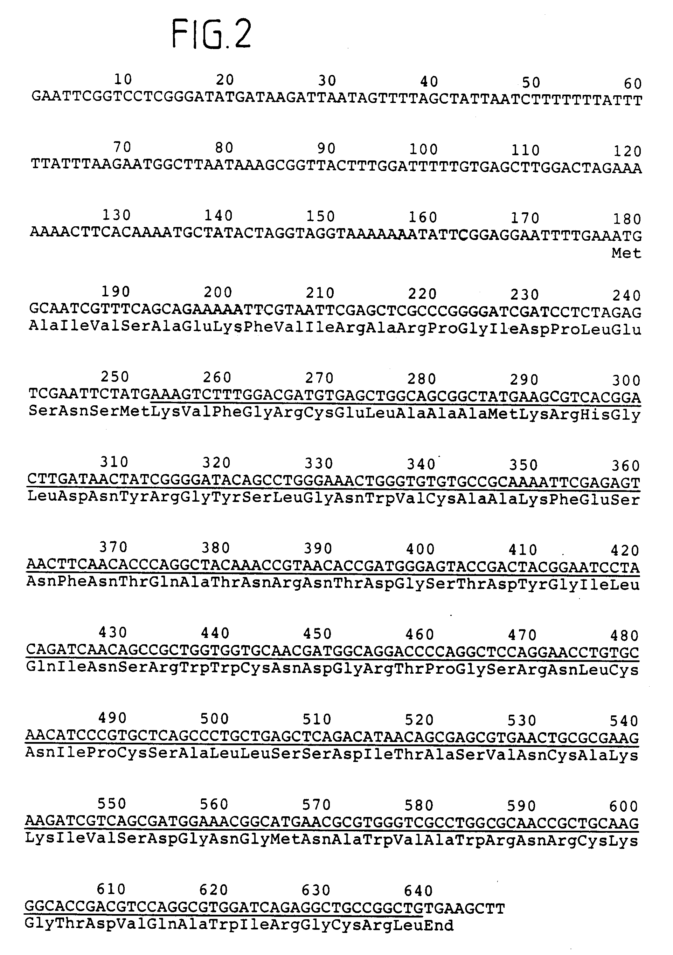

Fig. 2 shows the nucleotide sequence of an EcoRI-HindIII fragment containg the cDNA for the ripe hen's egg white lysozyme (the coding sequence is underlined). The EcoRI-HindIII 243-647 (numbering as used in Fig. 2) originates from plasmid pKLZ61 (Imoto et al., Protein Engineering 1, 1987, 333-338). Fragment 1-242 originates from pGKV432.

### Extensive description of the invention and preferred embodiments thereof

This invention provides lactic acid bacteria having a property they naturally lack, namely, the power of producing an active lysozyme type enzyme which enables them during use in the manufacture of cheese to inhibit the occurrence of butyric acid fermentation. For practical and economical reasons it is attractive to have strains of lactic acid bacteria available that are inherently capable of controlling Clostridium tyrobutyricum infections during cheese ripening, so that a separate addition of lysozyme is superfluous and the addition of nitrate can be reduced or even fully omitted.

The genetic manipulation according to the invention is in principle not limited to one specific species of lactic acid bacteria. The recombinant vectors and plasmids described in the practical part are useful in lactic acid bacteria of divergent species, such as streptococci, lactococci and lactobacilli, and therefore not only in the species Lactococcus lactis subsp.lactis (strain IL1403) used in the practical part. If necessary, adapted vectors are used for a genetic manipulation of other types of lactic acid bacteria, which a skilled worker is to be deemed capable of, on the basis of his normal expert knowledge and the information given therein.

Although, in principle, a genetic manipulation is also possible in which the information required for the power of producing a lysozyme is included in the genome (the chromosomes) of the lactic acid bacteria, the formation elucidated in the practical part of genetically manipulated lactic acid bacteria having a plasmid encoding power of producing a lysozyme is preferred because the property can then be more easily transferred to other strains.

As regards the lysozyme to be expressed, the invention is, in principle, not limited either. Any lysozyme or lysozyme-like enzyme capable of inhibiting the growth of butyric acid bacteria can be used. But the examples of useful lysozymes as elucidated in the practical part are preferred, because the DNA coding for these enzymes is cloned and well characterized. These enzymes are the hen's egg white lysozyme and the R gene product of bacteriophage lambda. Further information on these enzymes and the genetic information coding therefor can be found in the literature, including the literature mentioned in the chapter "Background of the invention". The operating mechanism of the lambda R gene product shows strong similarities with that of hen's egg white lysozyme, although there are clear structural differences between both enzymes. As appears from the practical part, the genetic information coding for these enzymes is generally accessible via known plasmids.

By means of DNA recombinant techniques the genetic information coding for a lysozyme may be incorporated in suitable expression vectors,in particular plasmids capable of replication in lactic acid bacteria and having a number of features enabling them to be used as vectors. Different cloning vectors suitable for lactic acid bacteria are known. Preferably, however, use is made of a vector containing (like plasmid pGK11) the replication origin of plasmid pWV01. The point is that this replication origin has the attractive property of functioning not only in lactic acid bacteria (pWV01 is a plasmid of the lactic acid bacterium Lactococcus lactis subsp.cremoris Wg2), but also in Escherichia coli and Bacillus subtilis. The pWV01 origin is publicly accessible by means of the natural plasmid pWV01 itself or by means of construct pGK11, both described by Kok et al. in Appl.Environ.Microbiol.48, 1984, 726-731.

In order to be able to function as expression vector, the plasmid must have not only a suitable replication origin but also expression signals active in lactic acid bacteria. Suitable expression signals have already been published, such as the expression signals described by Kok et al. in Appl.Environ.Microbiol.54, 1988, 231-238 (promoter and signal sequence, terminator sequence) of the proteinase gene located on plasmid pWV05 of the above-mentioned Wg2 strain. These nucleotide sequences are publicly accessible via the natural plasmid pWV05 itself or via known constructs, such as plasmid pGKV512, described by Kok et al. in Appl.Environ.Microbiol.54, 1988, 239-244. Instead of the pWV05 proteinase gene promoter the promoter described by Van der Vossen et al. in Appl.Environ. Microbiol.53, 1987, 2452-2457, originating from chromosomal DNA of the Wg2 strain, may also be used. Together with a ribosomal binding site and the start of an open reading frame, this promoter is accessible via plasmid pGKV232. Because there is no signal sequence, using this promoter will result in that under control of this promoter expressed lysozyme is not separated by the lactic acid bacteria. However, other promoters and terminator sequences are also useful in principle, as long as they function in lactic acid bacteria. Consequently, this invention is not limited to the expression signals used in the practical part.

As will be clear to a skilled worker, expression vectors containing a multiple cloning site (MCS) are preferred. Through the presence of an MCS the construction of plasmids according to the invention is facilitated and, in particular, the correct reading frame can be easily ensured. Suitable MCS sequences are known per se and are accessible, e.g., via the publicly available pUC plasmids, such as pUC18. The MCS of pUC18 contains, inter alia, SalI, AccI and HindII sites enabling a selected gene to be inserted in the correct reading frame. After these sites have been filled into blunt ends by means of Klenow enzyme, fragments with blunt ends can be inserted in three reading frames on sites positioned at mutual distances of 1 base pair.

Similarly, it will be clear that suitable vectors usually contain at least one selectable feature to enable successful constructions or successful transformations (transformants) to be distinguished from unsuccessful constructions and transformations. It is very conventional to use antibiotic resistance genes for this purpose. In the meantime, a good many of them have been known. As examples of useful antibiotic resistance genes the kanamycin resistance gene of Streptococcus faecalis plasmid pJH1 (described by Trieu-Cuot and Courvalin in Gene 23, 1983, 331-341) and the erythromycin resistance gene of Streptococcus aureus plasmid pE194 (described by Weisblum et al., J.Bacteriol.137, 1979, 635-643) have been selected in the practical part. Both are publicly accessible and isolated according to the practical part from plasmids pPJ1 (a pUC7 plasmid containing the Km^{r} gene of pJH1) and pUC7e (a pUC7 plasmid containing Em^{r} gene of pE194). The kanamycin resistance gene (Km^{r} gene) has the advantage of an easy selection in E.coli and in B.subtilis, but selection in L.lactis proves more difficult owing to the occurrence of a high background of non-transformed cells, also when instead of kanamycin neomycin is used as selective antibiotic. Since selection for erythromycin resistance in L.lactis is much more efficient, the use of expression vectors containing the erythromycin resistance gene (Em^{r}) is preferred.

In addition to antibiotic resistance genes, there are also other selectable features. In the present case dealing with genetically manipulated lactic acid bacteria for use in the manufacture of cheese a selectable feature originating from lactic acid bacteria is preferred over selection features that do not originate from lactic acid bacteria.

As shown in the practical part, there can be constructed plasmids leading to the production of a fusion protein, i.e. a protein containing redundant sequences in addition to the amino acid sequence of the lysozyme. These additional sequences may have a disadvantageous effect on the activity of the lysozyme type enzyme. For this reason it is preferred that redundant nucleotide sequences coding for these redundant and possibly disturbing amino acids are removed. This removal of redundant nucleotide sequences can be realized by means of a known site directed mutation method (site directed mutagenesis), such as the gapped duplex DNA method described by Kramer et al. in Nucl.Acids Res.12, 1984, 9441-9456, using the phasmids especially designed therefor (i.e. plasmid/phage hybrids) pMa5-8 and pMc5-8, described by Stanssens et al. in the manual entitled "Oligonucleotide-directed construction of mutations by the gapped duplex DNA method using the pMa/c phasmid vectors", November 1986, jointly edited by Plant Genetic Systems, Gand, Belgium, and Max Planck Institut für Biochemie, Martinsried, Federal Republic of Germany. The pMa5-8 contains an ampicillin resistance gene and a chloramphenicol resistance gene inactive owing to an amber mutation. The pMc5-8 is nearly equal to pMa5-8 but contains an active chloramphenicol resistance gene and an ampicillin resistance gene inactive owing to an amber mutation. As shown in the practical part, this method has been used in the construction of plasmids pMG37 and pMG38 which contain the gene coding for hen's egg white lysozyme and in the construction of plasmids pMG41 and pMG42 containing the lambda R gene. In case of the construction of plasmid pMG37 the primer used in this site directed mutation consisted of single-stranded DNA with the sequence (in the 5′-3′ direction)
GCTCACATCGTCCAAAGACTTTCATTTCAAAATTCCTCCGAATA.
This synthetic oligonucleotide consisting of 44 nucleotides is complementary to the nucleotides (according to the numbering of Fig. 2) 159-180 [originating from promoter P32]and 253-274 [ originating from the EcoRI-HindIII fragment of plasmid pKLZ61 (described by Imoto et al. in Protein Engineering 1, 1987, 333-338), in which the sequence coding for hen's egg white lysozyme is located]. The use of this primer resulted in a removal of the redundant nucleotides 181-252. In the construction of plasmid pMG38 the primer used was a single-stranded DNA with the sequence (in 5′-3′ direction)
GCTCACATCGTCCAAAGACTTTCGCCTTTGCTTGGATTTCGCC.
In case of plasmids with bacteriophage lambda R gene such a site directed mutation is likewise necessary, because in the pAP19 fragment used as a source for the R gene (a construct obtained by insertion of the HinfI fragment containing lambda R gene (nucleotides 45439-45976) in the EcoRV site of plasmid pIC19H described by Marsh et al. in Gene 32, 1984, 481-485) before the R gene a stop codon occurs in the same reading frame as the R gene.
In the construction of plasmid pMG41 the primer used was a single-stranded DNA with the sequence (in the 5′-3′ direction)
CGTTGATTATTGATTTCTACCATTTCAAAATTCCTCCGAAT.
In the construction of plasmid pMG42 the primer used was a single-stranded DNA with the sequence (in the 5′-3′ direction)
TACGTTGATTATTGATTTCTACCGCCTTTGCTTGGATTTCG.

This invention is not limited to genetically manipulated lactic acid bacteria as defined and described above and to recombinant plasmids capable of being used for the relevant genetic manipulation but also extends to recombinant vectors from which the above plasmids have been derived by inserting the gene coding for a lysozyme. These vectors are in fact also very suitable for the expression of proteins other than lysozymes in lactic acid bacteria. Therefore, they can be used in cases of strain improvement (the formation of lactic acid bacteria having improved or even completely new properties) and in using lactic acid bacteria as a producer of heterologous proteins.

The invention will be explained in more detail by means of the following practical part, which is not meant to be restrictive.

### Practical part

### (a) Bacterial strains

### E.coli

- BHB2600 (Hohn, Methods Enzymol.68, 1979, 299-309)
- C600(Appleyard, Genetics 39, 1954, 440-452)
- JM103(Messing et al., Nucl.Ac.Res.9, 1981, 309-321)
- MC1000(Casadaban and Cohen, J.Mol.Biol.138, 1980, 179-207)
- WK6mutS(manual of Stanssens et al., 1986)

### B.subtilis

- PSL1(Ostroff and Pene, J.Bacteriol.156, 1983, 934-936)

### L.lactis subsp.lactis

- IL1403(Chopin et al., Plasmid 11, 1984, 260-263)

### (b) Plasmids

- pWV01 (Kok et al., Appl.Environ.Microbiol.48, 1984, 726-731; a Lactococcus lactis subsp.cremoris Wg2 plasmid, the origin of which also functions in E.coli and B.subtilis)
- pGK11 (Kok et al., Appl.Environ.Microbiol.48, 1984, 726-731; Cm^{r}; contains the origin of pWV01 on a Sau3A fragment)
- pWV05 (Kok et al., Appl.Environ.Microbiol.54, 1988, 231-244; a plasmid containing the complete Wg2 protease gene, including terminator);
- pJH1 (Trieu-Cuot and Courvalin, Gene 23, 1983, 331-341; Em^{r},Km^{r},Str^{r},Tc^{r}; a Streptococcus faecalis plasmid containing, inter alia, a kanamycin resistance gene)
- pGKV512 (Kok et al., Appl.Environ.Microbiol.54, 1988, 239-244)
- pGKV232 (Van der Vossen et al., Appl.Environ.Microbiol.53, 1987, 2452-2457; Cm^{r},Em^{r}; a plasmid containing a promoter P32 originating from chromosomal DNA of Lactococcus lactis subsp.cremoris Wg2 with ribosomal binding site and start of an open reading frame)
- pE194 (Horinouchi and Weisblum, J.Bacteriol.150, 1982, 804-814; Weisblum et al., J.Bacteriol.137, 1979, 635-643; Em^{r})
- pUC7 (Vieira and Messing, Gene 19, 1982, 259-268)
- pUC18 (Yanisch-Perron et al., Gene 33, 1985, 103-119; Ap^{r})
- M13mp18/19 (Yanisch-Perron et al., Gene 33, 1985, 103-119)
- pPJ1 (Ap^{r},Km^{r}; pUC7 having therein the kanamycin resistance gene of pJH1 on a HindII fragment)
- pUC18e (contains a multiple cloning site MCS having thereon in the BamHI site an erythromycin resistance gene)
- M13prot93 (phage M13mp18/19 with the pWV05 protease gene terminator in a 5 kb PstI fragment)
- pGKV514 (a plasmid obtained by cutting with AatII and religation of pGKV512, said plasmid containing the promoter of the protease gene of the Wg2 strain in a 500 bp NruI-HaeIII fragment)
- pGKV432 (Cm^{r},Em^{r}; a plasmid derived by cutting with BamHI, Klenow and religation of pGKV232, said plasmid containing a promoter P32 originating from chromosomal DNA of Lactococcus lactis subsp.cremoris Wg2 with ribosomal binding site and start of an open reading frame in an EcoRI-SalI fragment)
- pUC7e (Ap^{r},Em^{r}; pUC7 having therein the erythromycin resistance gene of pE194 in an AccI fragment)
- pMG6 (contains a PstI-HindIII fragment having therein a lysozyme lacZ fusion sequence, said fragment being composed of 3 partial fragments which are ligated with each other after the formation of blunt ends; these partial fragments are successively (1) a PstI-SacI fragment (5′ part of the lysozyme sequence) from pLS1023; (2) an EcoRI-DraI fragment (lacZ fragment) from pMLB1034, from which the BamHI site had been removed previously by means of Mung bean nuclease; and (3) a ClaI-HindIII fragment from pBR322)
- pMG18 (contains a PstI-HindIII fragment having therein a lysozyme cDNA sequence, said fragment being composed of 2 partial fragments which are ligated with each other after the formation of blunt ends; these partial fragments are successively (1) a PstI-DraI fragment (lysozyme fragment) from pLS1023; and (2) an EcoRI-HindIII fragment from pBR322)
- pLS1023 (Land et al., Methods Enzymol., 100, 1983, 285-292)
- pMLB1034 (Silhavy et al., "Experiments with gene fusions", 1984, Cold Spring Harbour Laboratory, Cold Spring Harbour, New York)
- pBR322 (Bolivar et al., Gene 2, 1977, 95-113)
- pKLZ61 (Imoto et al., Protein Engineering 1, 1987, 333-338; Ap^{r}; contains hen's egg white lysozyme cDNA)
- pICI9H (Marsh et al., Gene 32, 1984, 481-485)
- pAP19 (pICI9H having in the EcoRV site a HinfI fragment originating from phage lambda (nucleotides 45439-45976), which contains the lambda R gene)
- pMa6-8 (Ap^{r},Cm^{s}) and pMc5-8 (Ap^{s},Cm^{r}), Stanssens et al., manual 1986)

### (c) Constructs

pMG20; pMG21; pMG23; pMG25; pMG27; pMG29; pMG30; pMG32; pMG32delta; pMG33a; pMG33; pMG36 (ca. 3.7 kb); pMG36e (ca. 3.7 kb); pMG36HEL; pMG36eHEL; pMG37; pMG33e; pMG33eHEL; pMG38; pMG36lambdaR; pMG33lambdaR; pMG 41; and pMG42.

### (d) Culturing methods

E.coli and B.subtilis were cultured in TY broth (Rottlander and Trautner, J.Mol.Biol.108, 1970, 47-60) or on TY solidified with 1.5% agar. For transformation of B.subtilis protoplasts the plating medium used was DM3 (Chang and Cohen, Mol.Gen.Genet.168, 1979, 111-115). For culturing L.lactis use was made of glucose M17 broth (Terzaghi and Sandine, Appl.Microbiol.29, 1975, 807-813) or glucose M17 solidified with 1.5% agar. In order to osmotically stabilize cells subjected to electroporation, 0.3 M sucrose was added to these media.
Kanamycin was used in concentrations of 50 and 20 ug/ml for E.coli and B.subtilis, respectively, with the understanding that 100 ug/ml was used for DM3 plates. Erythromycin was used in concentrations of 100 and 5 ug/ml for E.coli and L.lactis, respectively.

### (e)Isolation of plasmid DNA, restriction enzyme analysis and cloning of genes

Plasmid DNA was isolated in essence by the method of Birnboim and Doly (Nucl.Acids Res.7, 1979, 1513-1523). Restriction enzymes, Klenow enzyme and T4 DNA ligase were supplied by Boehringer Mannheim GmbH and used as published. For the transformation of E.coli the method of Mandel and Higa (J.Mol.Biol.53, 1970, 159-162) was used. Protoplasts of B.subtilis were prepared and transformed by the procedure of Chang and Cohen (Mol.Gen.Genet.168, 1979, 111-115). Plasmids were brought into L.lactis by electroporation (Van der Lelie et al., Appl.Environ.Microbiol.54, 1988, 865-871).

### (f) Construction of the expression vectors

The various steps of the performed constructions are described hereinbelow. Relevant information on the plasmids is given in brackets. The term "klenow" means that a treatment with Klenow DNA polymerase was conducted to form blunt ends.
1. Construction of pMG20 (Km^{r}, ori pWV01)
   1a. pGK11 (ori pWV01)+Sau3A,klenow
   1b. pPJ1(Km)+HindII
   1c. ligation, transformation of E.coli MC1000, selection Km₂₀^{R}, Cm₅^{S}, Ap₁₀₀^{S}
2. Construction of pMG21 (removal of the two HindIII sites in the pPJ1 part)
   2a. pMG20+HindIII,klenow
   2b. ligation, transformation of B.subtilis PSL1 protoplasts, selection Km₁₀₀^{R}(DM3 medium)
3. Construction of pMG23 (Km^{r}, ori pWV01, Em^{r} in MCS)
   3a. pMG21+ClaI,klenow
   3b. pUC18e+EcoRI,HindIII,klenow
   3c. ligation, transformation of E.coli C600, selection Km₅₀Em₅₀^{R}, AP50^{S}
4. Construction of pMG25 (removal of the Em^{r} gene from the MCS)
   4a. pMG23+BaMHI
   4b. ligation, transformation of E.coli C600, selection Km₅₀^{R}, Km₅₀Em₅₀^{S}
5. Construction of pMG27 (Km^{r}, ori pWV01, terminator in MCS)
   5a. pMG25+SphI,T4 DNA polymerase+dNTPs,PstI, isolation vector part
   5b. M13prot93+PstI,AccI, isolation 1021 bp fragment, FnuDII,HaeII
   5c. ligation, transformation of E.coli C600, selection KM₅₀^{R}
6. Construction of pMG29
   6a. pMG27+PstI,HindIII, isolation vector part
   6b. pMG6+PstI,HindIII,isolation lysozyme lacZ fusion fragment
   6c. ligation, transformation of E.coli BHB2600, selection Km₅₀^{R}
7. Construction of pMG30 (Km^{r}, ori pWV01, protease gene promoter and terminator in MCS)
   7a. pMG29 +BamHI,SalI,klenow
   7b. pGKV514 +NruI,HaeIII,isolation 500 bp promoter fragment
   7c. ligation, transformation of E.coli MC1000, selection Km₅₀^{R}, Xgal₄₀ blue
8. Construction of pMG32
   8a. pMG30 +PstI,HindIII
   8b. pMG18+PstI,HindIII
   8c. ligation, transformation of E.coli MC1000, selection KM₅₀^{R}, Xgal₄₀ white, Tc_{12,5}^{S}
9. Construction of pMG32delta (removal of the SmaI and BamHI sites)
   9a. pMG32+SmaI,BamHI,klenow
   9b. ligation, transformation of E.coli MC1000, selection Km₅₀^{R}
10. Construction of pMG33a (introduction of MCS and Em^{r} behind the protease gene promoter)
   10a. pMG32delta+HindII,HindIII
   10b. pUC18e+EcoRI,klenow,HindIII
   10c. ligation, transformation of E.coli C600, selection Km₅₀Em₅₀^{R}
11. Construction of pMG33 (removal of the Em^{r} gene)
   11a. pMG33a+BamHI
   11b. ligation, transformation of E.coli MC1000, selection Km₅₀R,Em₅₀^{S}
12. Construction of pMG36 (Km^{r}, Wg2 promoter P32, MCS, terminator, ori pWV01; expression vector pMG36 is shown in Fig. 1)
   12a. pMG33+EcoRI,SalI
   12b. pGKV432+EcoRI,SalI
   12c. ligation, BamHI, transformation of E.coli BHB2600, selection Km₅₀^{R}
13. Construction of pMG36e (Em^{r}, Wg2 promoter P32, MCS, terminator, ori pWV01)
   13a. pMG36+EcoRV
   13b. pUC7e+AccI,klenow
   13c. ligation, transformation of E.coli C600, selection Em₁₀₀^{R},Km₅₀^{S}
14. Construction of pMG36HEL (pMG36 + hen's egg white lysozyme gene)
   14a. pMG36+AccI,klenow,HindIII
   14b. pKLZ61+EcoRI,klenow,HindIII (the sequence of the EcoRI-HindIII fragment of pKLZ61 is shown in Fig. 2 from number 243)
   14c. ligation, transformation of E.coli, selection Km₅₀^{R}
15. Construction of pMG36eHEL (pMG36e + hen's egg white lysozyme gene)
   15a. pMG36HEL+EcoRV, isolation vector part
   15b. pUC7e+AccI,klenow,isolation of 1 kb fragment
   15c. ligation, transformation of E.coli BHB2600, selection Em₁₀₀^{R}
16. Construction of pMG37 (removal of redundant sequences from pMG36HEL)
   16a. pMG36HEL+EcoRV,HindIII, isolation of the promoter and lysozyme gene-containing fragment
   16b. pMa5-8+SmaI,HindIII
   16c. ligation, etc., followed by isolation of single-stranded DNA
   16d. pMc5-8+EcoRI,HindIII
   16e. gapped duplex forming
   16f. addition of the single-stranded oligonucleotide (5′ to 3′)
      GCTCACATCGTCCAAAGACTTTCATTTCAAAATTCCTCCGAATA
   16g. in vitro filling and ligation
   16h. transformation of E.coli WK6MutS, selection Cm^{R}
   16i. plasmid DNA isolation, SmaI, transformation of E.coli WK6
   16j. plasmid DNA isolation, restriction enzyme analysis, sequence analysis
   16k. isolation of the EcoRI-HindIII fragment and introduction thereof into pMG36e/ EcoRI,HindIII
17. Construction of pMG33e (Em^{r}, protease gene promoter, MCS, terminator, ori PWV01)
   17a. pMG33+EcoRV
   17b. pUC7e+AccI,klenow
   17c. ligation, transformation of E.coli C600, selection Em₁₀₀^{R}, Km₅₀^{S}
18. Construction of pMG33eHEL (pMG33e + hen's egg white lysozyme gene)
   18a. pMG33e+XbaI,HindIII
   18b. pMG36HEL+XbaI,HindIII
   18c. ligation, transformation of E.coli BHB2600, selection Em₁₀₀^{R}
19. Construction of pMG38 (removal of redundant sequences from pMG33eHEL)
   19a. pMG33eHEL+SspI,HindIII, isolation of the fragment containing the signal sequence and the sequence coding for lysozyme
   19b. pMa5-8+SmaI,HindIII
   19c. ligation, etc. followed by isolation of single-stranded DNA
   19d. pMc5-8+EcoRI,HindIII
   19e. gapped duplex forming
   19f. addition of the single-stranded oligonucleotide (5′ to 3′)
      GCTCACATCGTCCAAAGACTTTCGCCTTTGCTTGGATTTCGCC
   19g. in vitro filling and ligation
   19h. transformation of E.coli WK6MutS, selection Cm^{R}
   19i. plasmid DNA isolation, SmaI, transformation of E.coli WK6
   19j. plasmid DNA isolation, restriction enzyme analysis, sequence analysis
   19k. isolation of the EcoRI-HindIII fragment and introduction thereof into pMG33e/ EcoRI,HindIII
20. Construction of pMG36lambdaR (pMG36 + lamda R gene)
   20a. pMG36+SacI,SalI
   20b. pAP19+SacI,SalI
   20c. ligation, transformation of E.coli MC1000, selection Km₅₀^{R}
21. Construction of pMG33lambdaR (pMG33 + lambda R gene)
   21a. pMG33+BamHI,SalI
   21b. pAP19+BglII,SalI
   21. ligation, transformation of E.coli MC1000, selection Km₅₀^{R}
22. Construction of pMG41 (removal of redundant sequences from pMG36lambdaR)
   A site directed mutagenesis was carried out analogously to the procedure described under item 16 but applied to the plasmid pMG36lambdaR and using the primer:
   CGTTGATTATTGATTTCTACCATTTCAAAATTCCTCCGAAT
23. Construction of pMG42 (removal of redundant sequences from pMG33lambdaR)
   A site directed mutagenesis was carried out analogously to the procedure described under item 16 but applied to the plasmid pMG33lambdaR and using the primer:
   TACGTTGATTATTGATTTCTACCGCCTTTGCTTGGATTTCG

### (g) Gene product analysis

In vitro transcription and translation were carried out by means of the translation kit for prokaryotic DNA of Amersham International plc, Amersham, England.
Lysates of L.lactis for Western blot analysis were prepared according to the following protocol: the strains were cultured overnight in glucose M17 broth, supplemented with 40 mM DL-threonine. Of this culture samples of 10 ml were centrifuged.The cell pellet was washed with 10 ml STE (100 mM NaCl, 10 mM Tris-HCl pH 7, 1 mM EDTA), resuspended in 1 ml STE and transferred into an Eppendorf tube. After recentrifuging the cells the pellet was resuspended in 50 ul of a 10 mM Tris-HCl, 1 mM EDTA solution (pH 7), which contained 150 U/ml mutanolysin and 1 mM MgCl₂. After 30 minutes of incubation at 37°C there was added one volume amount of twice filled-in buffer for polyacrylamide gel electrophoresis, followed by heating the samples for 10 min at 100°C. Cell residues were removed by centrifugation, and portions of mostly 1/10 of the supernatant were used for further analysis.
Products of in vitro transcription-translation and cell lysates were applied to 15% SDS polyacrylamide gels (Laemmli, Nature 227, 1970, 680-685) and subjected to electrophoresis. Products of in vitro transcription-translation were then visualized by autoradiography. The electrophoretically separated proteins from the cell lysates were applied to nitrocellulose by electroblotting, followed by detecting hen's egg white lysozyme sequences with a rabbit antiserum against hen's egg white lysozyme and a peroxidase-conjugated hog antiserum against rabbit immunoglobulins (Dakopatts a/s, Glostrup, Denmark).
In order to examine whether the enzyme produced in L.lactis is present in active form, cell lysates were prepared by sonication at 0°C (6 cycles of 20 sec with intervals of 20 sec, amplitude 4 micrometer, in a Soniprep 150 of MSE Ltd., Sussex, England). After removal of cell residues by centrifugation portions of the supernatant were added to a suspension of lyophilized Micrococcus lysodeikticus cells (Sigma, St.Louis, USA), and the Δ A450 representing the lysozyme activity was measured as prescribed.

### (h) Description of the experiments and the results obtained

As noted above under (f), different new plasmids were constructed. There were first constructed suitable expression vectors, namely, pMG33, pMG33e, pMG36 and pMG36e. pMG36 is shown in Fig. 1. This expression vector is based, inter alia, on the L.lactis subsp.cremoris Wg2 plasmid pWV01, which also replicates in E.coli and in B.subtilis. The part of the vector containing the pWV01 replication origin was isolated from pGK11 by cutting with Sau3A and filling the sticky ends by means of Klenow enzyme. The kanamycin resistance marker originating from the S.faecalis plasmid pJH1, was isolated from plasmid pPJ1 as a HindII fragment. These two fragments were ligated, and the ligation mixture was used for transforming E.coli MC1000 with selection for kanamycin resistance. Subsequently, two HindIII sites located in the terminator region of the kanamycin resistance gene were removed by cutting with HindIII, filling the sticky ends by means of Klenow enzyme, religating and transforming protoplasts of B.subtilis. The resulting plasmid (pMG21) contained a unique ClaI site used for introducing the MCS of pUC18 and the transcription terminator sequence of the proteinase gene of the Wg2 strain. In fact, two fragments of the proteinase gene and the adjacent sequences were cloned: a HindIII-HaeII fragment (nucleotides 6532-6651) and a HaeII-FnuDII fragment (nucleotides 6970-7141), which contained the terminator sequence. These and the following steps in the vector construction were carried out with E.coli as a host for cloning. Finally, part of the MCS was replaced by an EcoRI-SalI fragment from pGKV432, which contained a promoter, a ribosome binding site and the start of an open reading frame, originating from chromosomal DNA of the Wg2 strain. It should be noted that the HindIII site in the MCS of pMG36 is unique because of the removal of the HindIII sites in the terminator region of the kanamycin resistance gene.

The expression vector pMG36 was used as starting-point for the construction of, inter alia, recombinant plasmids pMG36HEL, pMG37, pMG36lambdaR, and pMG41. From plasmid pKLZ61 an EcoRI-HindIII fragment of 400 bp was isolated which contained the sequence coding for ripe hen's egg white lysozyme. This fragment, the sequence of which is known, was brought into pMG36. Upstream of the hen's egg white lysozyme gene, the sticky EcoRI end was suitably filled by means of Klenow enzyme and ligated in the AccI site of pMG36, which was also blunted first by means of Klenow enzyme. Sticky HindIII end immediately located downstream of the stop codon of the lysozyme gene was ligated in the unique HindIII site of pMG36. The reappearance of an EcoRI site at the location of the AccI-EcoRI fusion made it possible to check whether the construction had proceeded correctly. The resulting plasmid was called pMG36HEL. This plasmid pMG36HEL was subjected to in vitro transciption and translation in an E.coli system, followed by SDS polyacrylamide gel electrophoresis. This resulted in binding a protein band of approximately 17 kDa, which was absent in a control lane on the basis of pMG36 (results not shown). The size of the protein corresponds well to the size of the expected fusion protein (the expected molecular weight of the fusion protein was 17.0 kDa; hen's egg white lysozyme has, for comparison, a molecular weight of 14.3 kDa).

Because it was found that in L.lactis selection for erythromycin resistance is more efficient than selection for kanamycin or neomycin resistance, the greater part of the kanamycin resistance gene was removed from pMG36HEL by treatment with EcoRV and replaced with a 1 kb fragment from pUC7e, which contained the erythromycin resistance gene of the S.aureus plasmid pE194. The resulting plasmid pMG36eHEL was transferred by electroporation to L.lactis subsp.lactis strain IL1403, with selection for erythromycin resistance. In the same way the kanamycin resistance gene of pMG36 was replaced with the erythromycin resistance gene to obtain pMG36e. This plasmid was also transferred to L.lactis subsp. lactis strain IL1403.

Lysates of L.lactis (pMG36e) and L.lactis (pMG36eHEL) were prepared in the manner described under (g). SDS polyacrylamide gel electrophoresis was followed by a Western blot analysis by means of a rabbit antiserum against hen's egg white lysozyme. The results (not shown here) indicated the presence of a 17 kDa protein in L.lactis (pMG36eHEL) which did not occur in the strain containing pMG36e. This was a strong indication that the hen's egg white lysozyme fusion gene was expressed in L.lactis subsp.lactis IL1403.

Lysates of the two plasmid-containing L.lactis strains were also incubated with a suspension of Micrococcus lysodeikticus, following the decrease in A450. In this examination no differences between the two strains (the strain with pMG36eHEL and the control strain with pMG36e) were observed, from which it had to be concluded that the fusion protein either was inactive or was formed in too slight an amount to be detected in this experiment.

The fusion gene (462 bp) comprises a 387 bp sequence coding for ripe lysozyme, preceded by the 5′ part of an unknown chromosomal gene of L.lactis subsp.cremoris Wg2 and by a small piece of MCS. To maximize the chance of success, i.e., the formation of a lactic acid bacterium using active lysozyme type enzyme and inhibiting the growth of butyric acid bacteria without killing itself, there were examined two alternatives in which different new constructs were realized. For these two alternatives, there were constructed both a plasmid giving rise to separation of the produced gene product from the cell and a plasmid that does not give rise to such a separation.

A first type of alternatives consisted of the construction of recombinant plasmids which no longer lead to a fusion protein but to the ripe hen's egg white lysozyme itself. From plasmid pMG36eHEL there was constructed plasmid pMG37 by means of site directed mutagenesis (through the site directed mutagenesis nucleotides 181-252 of the fragment shown in Fig. 2 were removed). In L.lactis subsp.lactis IL1403 this plasmid gives rise to the formation of the ripe form of the hen's egg white lysozyme. This protein is intracellularly produced and proves to actively inhibit the growth of Clostridium tyrobutyricum. From plasmid pMG33 plasmid pMG38 was constructed via pMG33e and pMG33eHEL. Plasmid pMG33eHEL contains the lysozyme cDNA sequence, preceded by the promoter and signal sequence of the protease gene of the Wg2 strain. The redundant sequences between signal sequence and the sequence coding for lysozyme were removed by site directed mutagenesis, analogously to the construction of pMG37. In L.lactis subsp.lactis IL1403 plasmid pMG38 gives rise to excretion of lysozyme which is active against butyric acid bacteria.

A second type of alternatives consisted of the replacement of the genetic information coding for hen's egg white lysozyme with the R gene of bacteriophage lambda which gives rise to the formation of a protein, the operating mechanism of which corresponds substantially to the hen's egg white lysozyme, but has a clearly different structure notwithstanding and probably shows a higher activity. Plasmid pMG41 constructed from pMG36 via pMG36lambdaR leads to intracellular production of active lambda R gene product. Plasmid pMG33 constructed from pMG33 via pMG33lambdaR results in separation of active lambda R gene product.

## Claims

1. Lactic acid bacteria, particularly of the species Lactococcus lactis, which as a result of a genetic manipulation have acquired the power of producing lysozyme.

2. Lactic acid bacteria as claimed in claim 1, which as a result of a genetic manipulation have acquired the power of producing an enzyme selected from hen's egg white lysozyme and the R gene product of bacteriophage lambda.

3. Lactic acid bacteria as claimed in claim 1 or 2, whose power of producing lysozyme is plasmid encoded.

4. Lactic acid bacteria as claimed in claim 3, containing at least one plasmid responsible for the power of producing lysozyme, said plasmid containing at least the replication origin of the Lactococcus lactis subsp.cremoris Wg2 plasmid pMV01 and a gene coding for a lysozyme under control of expression signals active in lactic acid bacteria.

5. The use of lactic acid bacteria as claimed in any of claims 1-4, in the manufacture of cheese.

6. A recombinant plasmid comprising a replication origin active in lactic acid bacteria and a gene coding for a lysozyme under control of expression signals active in lactic acid bacteria.

7. A recombinant plasmid as claimed in claim 6 comprising a replication origin active in lactic acid bacteria and either a gene coding for hen's egg white lysozyme or the R gene of bacteriophage lambda, under control of expression signals active in lactic acid bacteria.

8. A recombinant plasmid as claimed in claim 6 or 7, containing the replication origin of the Lactococcus lactis subsp.cremoris Wg2 plasmid pWV01.

9. A recombinant plasmid as claimed in any of claims 6-8, containing the terminator sequence of the proteinase gene of Lactococcus lactis subsp.cremoris Wg2.

10. A recombinant plasmid as claimed in any of claims 6-9, containing the promoter and the signal sequence of the protease gene of Lactococcus lactis subsp.cremoris Wg2.

11. A recombinant plasmid as claimed in any of claims 6-9, containing the promoter P32 originating from chromosomal DNA of Lactococcus lactis subsp.cremoris Wg2.

12. A recombinant plasmid as claimed in any of claims 6-11, which also contains one or more selection genes.

13. A recombinant plasmid as claimed in claim 12, which contains a kanamycin resistance gene of an erythromycin resistance gene.

14. A recombinant vector from which a recombinant plasmid as claimed in any of claims 6-13 is derived by inserting the gene coding for a lysozyme, such as the gene coding for hen's egg white lysozyme or the R gene of bacteriophage lambda.

## Patentansprüche

1. Milchsäurebakterien, inbesondere der Spezies Lactococcus lactis, die infolge einer genetischen Modifikation die Fähigkeit erworben haben, Lysozym zu produzieren.

2. Milchsäurebakterien nach Anspruch 1, die infolge einer genetischen Modifikation die Fähigkeit erworben haben, ein aus Hühnereiweißlysozym und dem R-Genprodukt des Bakteriophagen Lambda gewähltes Enzym zu produzieren.

3. Milchsäurebakterien nach Anspruch 1 oder 2, deren Fähigkeit, Lysozym zu produzieren, plasmidkodiert ist.

4. Milchsäurebakterien nach Anspruch 3, in denen mindestens ein Plasmid enthalten ist, das für die Fähigkeit, Lysozym zu produzieren, verantwortlich ist, wobei dieses Plasmid mindestens den Replikationsursprung des Lactococcus lactis subsp.cremoris Wg2-Plasmids pMV01 und ein für ein Lysozym kodierendes Gen unter Kontrolle von in Milchsäurebakterien wirksamen Expressionssignalen enthält.

5. Verwendung von Milchsäurebakterien nach einem der Ansprüche 1-4 in der Käseherstellung.

6. Rekombinantes Plasmid, enthaltend einen in Milchsäurebakterien wirksamen Replikationsursprung und ein für ein Lysozym kodierendes Gen unter Kontrolle von in Milchsäurebakterien wirksamen Expressionssignalen.

7. Rekombinantes Plasmid nach Anspruch 6, enthaltend einen in Milchsäurebakterien wirksamen Replikationsursprung und entweder ein für Hühnereiweißlysozym kodierendes Gen oder das R-Gen des Bakteriophagen Lambda unter Kontrolle von in Milchsäurebakterien wirksamen Expressionssignalen.

8. Rekombinantes Plasmid nach Anspruch 6 oder 7, das den Replikationsursprung des Lactococcus lactis subsp.cremoris Wg2-Plasmids pWV01 enthält.

9. Rekombinantes Plasmid nach einem der Ansprüche 6-8, das die Terminatorsequenz des Proteinasegens von Lactococcus lactis subsp.cremoris Wg2 enthält.

10. Rekombinantes Plasmid nach einem der Ansprüche 6-9, das den Promoter und die Terminatorsequenz des Proteinasegens von Lactococcus lactis subsp.cremoris Wg2 enthält.

11. Rekombinantes Plasmid nach einem der Ansprüche 6-9, das den aus chromosomaler DNS von Lactococcus lactis subsp.cremoris Wg2 stammenden Promoter P32 enthält.

12. Rekombinantes Plasmid nach einem der Ansprüche 6-11, das auch ein oder mehrere Selektionsgene enthält.

13. Rekombinantes Plasmid nach Anspruch 12, das ein Kanamycinresistenzgen oder ein Erythromycinresistenzgen enthält.

14. Rekombinanter Vektor, von dem ein rekombinantes Plasmid nach einem der Ansprüche 6-13 abgeleitet ist durch Insertion des für ein Lysozym kodierenden Gens, wie das für Hühnereiweißlysozym kodierende Gen oder das R-Gen des Bakteriophagen Lambda.

## Revendications

1. Des bactéries de l'acide lactique, et plus particulièrement de l'espèce Lactococcus lactis, ayant acquis à la suite d'une manipulation génétique le pouvoir de produire des lysozymes.

2. Des bactéries de l'acide lactique selon la revendication 1, ayant acquis à la suite d'une manipulation génétique le pouvoir de produire un enzyme choisi dans le lysozyme blanc des oeufs de poule, et le produit du gène R du bactériophage lambda.

3. Des bactéries de l'acide lactique selon la revendication 1 ou 2, dans lesquelles le pouvoir de produire des lysozymes est encodé sous forme de plasmide.

4. Des bactéries de l'acide lactique selon la revendication 3, comportant au moins un plasmide responsable de la production de lysozyme, ce plasmide contenant au moins l'origine de duplication du plasmide pMV01 du Lactococcus lactis subsp. cremoris Wg2, et un gène codant un lysozyme sous contrôle de signaux d'expression actifs dans les bactéries de l'acide lactique.

5. L'emploi de bactéries de l'acide lactique selon l'une quelconque des revendications 1 à 4, pour la production de fromage.

6. Un plasmide recombinant, comportant une origine de duplication active dans les bactéries de l'acide lactique, et un gène codant un lysozyme sous contrôle de signaux d'expression actifs dans les bactéries de l'acide lactique.

7. Un plasmide recombinant selon la revendication 6, comportant une origine de duplication active dans les bactéries de l'acide lactique, et soit un gène codant le lysozyme blanc des oeufs de poule, soit le gène R du bactériophage lambda, sous contrôle de signaux d'expression actifs dans les bactéries de l'acide lactique.

8. Un plasmide recombinant selon les revendications 6 ou 7, comportant l'origine de duplication du plasmide pMV01 du Lactococcus lactis subsp. cremoris Wg2.

9. Un plasmide recombinant selon l'une quelconque des revendications 6 à 8, comportant la séquence de fin du gène de protéinase du Lactococcus lactis subsp. cremoris Wg2.

10. Un plasmide recombinant selon l'une quelconque des revendications 6 à 9, comportant le promoteur et la séquence de signaux du gène de protéinase du Lactococcus lactis subsp. cremoris Wg2.

11. Un plasmide recombinant selon l'une quelconque des revendications 6 à 9, comportant le promoteur P32 provenant le l'ADN chromosomique du Lactococcus lactis subsp. cremoris Wg2.

12. Un plasmide recombinant selon l'une quelconque des revendications 6 à 11, comportant également un ou plusieurs gènes de sélection.

13. Un plasmide recombinant selon la revendication 12, comportant le gène de résistance à la kanamycine d'un gène de résistance à l'érythrocine.

14. Un vecteur recombinant, dont un plasmide recombinant selon l'une quelconque des revendications 6 à 13 est dérivé, en insérant le gène codant un lysozyme, tel que le gène codant le lysozyme blanc des oeufs de poule ou le gène R du bactériophage lambda.
